# EUROPEAN PATENT APPLICATION

(11) **EP 2 327 714 A1**
(43) Date of publication of application: **01.06.2011**
(21) Application number: 09809762.9
(22) Date of filing: 07.08.2009
(51) Int. Cl.: C07K 7/08, A61K 38/00, A61K 39/00, A61P 31/16

(54) **PROPHYLACTIC OR THERAPEUTIC AGENT FOR INFLUENZA VIRUS INFECTION**

(30) Priority: 29.08.2008 JP 2008222062
(71) Applicant: Keio University, Tokyo 108-8345 (JP)
(72) Inventor: SATO, Toshinori, Yokohama-shi Kanagawa 223-8522 (JP); ISHIHARA, Goshi, Tokyo 157-0074 (JP)
(74) Representative: HOFFMANN EITLE
(86) International application number: PCT/JP2009/064012
(87) International publication number: WO 2010/024108

(57) **Abstract**

The present invention provides a self-assembly of a compound or of a pharmaceutically acceptable salt thereof, the compound being represented by formula (I)

**R¹-Gly LeuAla Met Ala Pro Ser Val Gly His ValArg Gln His Gly-R²** **(I)**

(wherein R¹ represents a linear or branched acyl group having 14 to 24 carbon atoms; R² represents OH, NH2, NR³R⁴, a substituted or unsubstituted alkoxy group, a substituted or unsubstituted aryloxy group, or a substituted or unsubstituted aralkyloxy group; and R³ and R⁴ independently represent a substituted or unsubstituted alkyl group, a substituted or unsubstituted aryl group, a substituted or unsubstituted aralkyl group, an alkoxy group, or a hydroxy group, provided that both R³ and R⁴ do not represent a hydroxy group or an alkoxy group simultaneously).

## Description

### Technical Field

The present invention relates to a substance having a prophylactic or therapeutic effect on influenza virus infection, and a prophylactic or therapeutic agent for influenza virus infection. The present invention further relates to a method for preventing or treating influenza virus infection (influenza).

### Background Art

Influenza viruses have, in their envelope membranes, two types of spike glycoproteins, hemagglutinin (HA) and sialidase (NA, neuraminidase), which play an important role in the establishment of viral infection and viral budding from a host cell, respectively.

Hemagglutinin, involved in the first step of influenza virus infection, has various subtypes, based on the diversity of amino acid sequences of the antigen-determining regions (A-E), which are highly mutatable. While amino acid sequence homology among hemagglutinin subtypes ranges from 25 to 75%, the so-called receptor binding pocket region, which binds to a receptor of a host cell, is comparatively less mutatable, and its three-dimensional structure is well-conserved (Non-patent Literature 1 (NPL 1)).

To prevent influenza virus infection, research has been undertaken to develop a vaccine that specifically binds to hemagglutinin that is contributory to the establishment of an infection, and thereby inhibits the function of hemagglutinin.

For example, Patent Literature 1 (PTL 1) discloses a peptide for preventing influenza virus infection, and particularly discloses a liposome preparation thereof; and the effects of the liposome preparation *in vitro* have been confirmed.

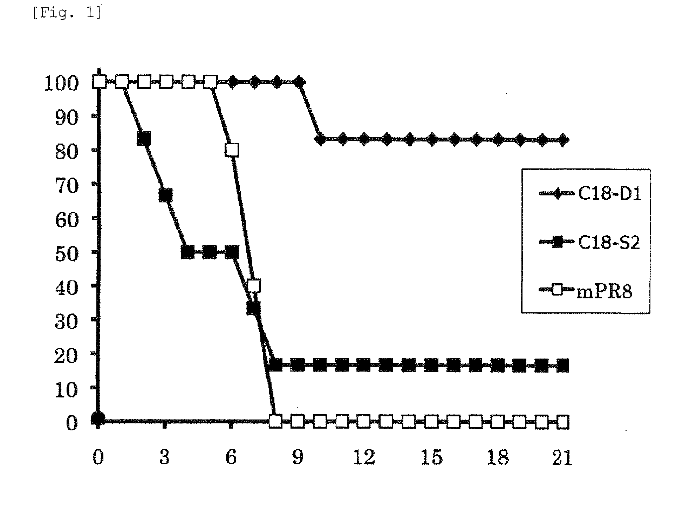

Patent Literature 2 (PTL 2) demonstrates in Fig. 1 that the peptide H3G-1 has an inhibitory effect on both H1N1 and H3N2. However, the inhibitory effect of the peptide H3G-1 was weak.

### Citation List

### Patent Literature

PTL 1: Japanese Unexamined Patent Publication No. 2002-284798
PTL 2: Japanese Unexamined Patent Publication No. 2006-101709

### Non-patent Literature

NPL 1: Y. Suzuki, Prog. Lipid. Res., 33, 429 (1994)

### Summary of Invention

### Technical Problem

An object of the present invention is to provide a substance that has a high prophylactic or therapeutic effect on influenza virus infection (influenza).

Another object of the present invention is to provide a prophylactic or therapeutic agent for influenza virus.

Another object of the present invention is to provide a method of preventing or treating influenza virus.

### Solution to Problem

The present inventors conducted further research on anti-influenza virus effects of a stearoylated peptide as set forth in SEQ ID NO: 1. As a result, the inventors found that the peptide in the form of a liposome preparation exerts an anti-influenza virus effect only on H3N2, whereas a self-assembly of the peptide has greater effectiveness for a wide range of influenza viruses, such as subtypes H1, H3, H5, and H7. The present inventors further found that the self-assembly of the peptide can be obtained by acylating the N-terminus of the peptide with an acyl group having 14 to 24 carbon atoms.

The present invention provides the following self-assembly and prophylactic or therapeutic agent for influenza virus infection.
Item 1. A self-assembly of a compound or of a pharmaceutically acceptable salt thereof,
   the compound being represented by the formula I:

   **R¹-Gly Leu Ala Met Ala Pro Ser Val Gly His Val Arg Gln His Gly-R²** **(I)**

   (wherein R¹ represents a linear or branched acyl group having 14 to 24 carbon atoms; R² represents OH, NH₂, NR³R⁴, a substituted or unsubstituted alkoxy group, a substituted or unsubstituted aryloxy group, or a substituted or unsubstituted aralkyloxy group; and R³ and R⁴ independently represent a substituted or unsubstituted alkyl group, a substituted or unsubstituted aryl group, a substituted or unsubstituted aralkyl group, an alkoxy group, or a hydroxy group; provided that both of R³ and R⁴ do not represent a hydroxy group or an alkoxy group simultaneously).
Item 2. The self-assembly according to Item 1 wherein R¹ represents a stearoyl group, a palmitoyl group, an oleoyl group, or a palmitoleyl.
Item 3. The self-assembly according to Item 1 wherein R² represents OH or NH₂.
Item 4. The self-assembly according to Item 1 wherein R¹ represents a stearoyl group, and R² represents OH or NH₂.
Item 5. The self-assembly according to any one of Items 1 to 4 wherein the self-assembly is in the form of a lyophilizate.
Item 6. A prophylactic or therapeutic agent for influenza virus infection comprising the self-assembly of any one of Items 1 to 5.
Item 7. The prophylactic or therapeutic agent according to Item 6 wherein the influenza virus is of subtype H1, H3, H5, or H7.
Item 8. The prophylactic or therapeutic agent according to Item 6 wherein the influenza virus is of subtype H1 or H3.
Item 9. The prophylactic or therapeutic agent according to Item 6 wherein the influenza virus is of subtype H1.
Item 10. A compound, a pharmaceutically acceptable salt thereof, or a self-assembly of the compound or of the pharmaceutically acceptable salt, the compound being represented by formula I:

   **R¹-Gly Leu Ala MetAla Pro Ser Val Gly His ValArg Gln His Gly-R²** **(I)**

   (wherein R¹ represents a stearoyl group, and R² represents NH₂). Item 11. A method of preventing or treating influenza virus infection, comprising administering an effective amount of a compound, a pharmaceutically acceptable salt thereof, or a self-assembly of the compound or of the pharmaceutically acceptable salt to a patient infected with an influenza virus or a subject who is at risk for infection,
   the compound being represented by formula I:

   **R¹-Gly Leu Ala MetAla Pro Ser Val Gly His ValArg Gln His Gly-R²** **(I)**

   (wherein R¹ represents a linear or branched acyl group having 14 to 24 carbon atoms; R² represents OH, NH₂, NR³R⁴, a substituted or unsubstituted alkoxy group, a substituted or unsubstituted aryloxy group, or a substituted or unsubstituted aralkyloxy group; and R³ and R⁴ independently represent a substituted or unsubstituted alkyl group, a substituted or unsubstituted aryl group, a substituted or unsubstituted aralkyl group, an alkoxy group, or a hydroxy group; provided that both of R³ and R⁴ do not represent a hydroxy group or an alkoxy group simultaneously).

### Advantageous Effects of Invention

According to the present invention, a potent prophylactic or therapeutic agent for influenza/influenza virus infection can be obtained.

The peptide set forth in SEQ ID NO: 2 exhibits a potent effect *in vitro*. However, this peptide is ineffective *in vivo,* and actually exacerbates influenza virus infection *in vivo.*

In contrast, the N-acylated peptides represented by formula (I) of the present invention, particularly the peptide set forth in SEQ ID NO: 2, exhibit a potent prophylactic or therapeutic effect on influenza viruses *in vivo* as well as *in vitro*.

Furthermore, C18D1 (stearoyl-GLAMAPSVGHVRQHG-NH2), when formed into a liposome, has a very low IC₅₀ value of about 500 nM against influenza viruses H1 and H3, and exhibits substantially no anti-influenza virus effect; whereas a self-assembly of C18D1 exhibits a highly potent effect.

### Brief Description of Drawings

[Fig. 1] Fig. 1 shows the survival benefit achieved by administering the peptide C18-D1 to influenza virus-infected mice. C18-D1 (C18-GLAMAPSVGHVRQHG-NH₂) and C18-S2 (C18-ARLPRTMVHPKPAQP-NH₂) were used. The dose of the peptide C18-D1 was 17.4 mg/(kg), and the dose of the peptide C18-S2 was 19.4 mg/(kg).

### Description of Embodiments

According to the present invention, a self-assembly of the compound represented by formula I shown below or a pharmaceutically acceptable salt thereof can be used as a prophylactic or therapeutic agent for influenza virus infection.

**R¹-Gly LeuAla MetAla Pro Ser Val Gly His ValArg Gln His Gly-R²** **(I)**

(wherein R¹ represents a linear or branched acyl group having 14 to 24 carbon atoms; R² represents OH, NH₂, NR³R⁴, a substituted or unsubstituted alkoxy group, a substituted or unsubstituted aryloxy group, or a substituted or unsubstituted aralkyloxy group; and R³ and R⁹ independently represent a substituted or unsubstituted alkyl group, a substituted or unsubstituted aryl group, a substituted or unsubstituted aralkyl group, an alkoxy group, or a hydroxy group; provided that both of R³ and R⁴ do not represent a hydroxy group or an alkoxy group simultaneously).

When the compound represented by formula I according to the present invention is formed into a self-assembly, the self-assembly exhibits an increased prophylactic or therapeutic effect on influenza virus infection. Furthermore, the self-assembly can gain effectiveness for a wide range of influenza viruses, such as subtype H1 (for example, H1N1, H1N2), subtype H3 (for example, H3N2), subtype H5 (for example, H5N1), and subtype H7 (for example, H7N1).

The effectiveness of the self-assembly of the present invention for subtypes H1 and H3 is demonstrated, as shown in the Examples below. Since the self-assembly of the compound represented by formula I is effective against both subtypes H1 and H3, the self-assembly is assumed to bind to a receptor-binding pocket region of a host cell, which is comparatively less mutatable and whose three-dimensional structure is well-conserved, thereby exhibiting an anti-influenza virus effect. Accordingly, the self-assembly of the compound represented by formula I according to the present invention is effective against infection of subtypes H1 and H3 as well as subtypes H5 and H7, which all have a common receptor-binding pocket region.

The self-assembly of the present invention is effective *in vivo* as well as *in vitro*. For example, C18-S2 (stearoyl ARLPRTMVHPKPAQP-NH₂), obtained by stearoylation of the N-terminus of S2 (ARLPRTMVHPKPAQP-NH₂), exhibits a potent anti-influenza virus effect *in vitro*. However, C18-S2 is ineffective *in vivo*, and actually causes early mortality due to influenza virus infection. Thus, the anti-influenza virus effect of C18-S2 is greatly different *in vivo* and *in vitro*. One feature of the peptide according to the present invention is that the peptide of the invention exhibits an anti-influenza virus effect *in vivo* as well as *in vitro*.

The self-assembly according to the present invention has a micelle-like structure wherein an acyl group moiety represented by R¹ is directed inward, and a peptide moiety is directed outward. Since a liposome preparation is in the form of a liposome containing a phospholipid as a major component, a self-assembly of the compound represented by formula I cannot be formed. Although Patent Literature 1 discloses an N-terminus-stearoylated (C18) peptide having a carboxyl group (COOH) on the C-terminus in the Examples, a stearoylated peptide having an amide (CONH₂) on the C-terminus according to the present invention is not disclosed therein.

Patent Literature 1 only discloses the production of a compound represented by formula I wherein R¹ represents a stearoyl group and R² represents OH, and a liposome preparation containing the compound. Patent Literature 1 does not disclose a self-assembly. While not wishing to be bound by theory, the present inventors believe that the effectiveness of the self-assembly of the compound of formula I according to the present invention for a wide range of influenza viruses is attributable to the structure of the self-assembly, because Patent Literature 1 demonstrates that the liposome preparation containing the compound of formula I is effective only for subtype H3, and is ineffective for subtype H1 (H1N1).

The N-terminus-non-acylated peptide of formula (I) (wherein R¹ is H), shown as H3G-1 in Fig. 1 of Patent Literature 2, exhibits an IC₅₀ value of > 100 µM in a plaque assay of the influenza virus H1N1, and thus has a very weak influenza virus infection-inhibitory effect. The inventors believe that the self-assembly structure is important because the peptide of formula (I) wherein R¹ is H cannot form a self-assembly.

The self-assembly of the compound of formula I can be produced by dissolving the compound of formula I in water, optionally followed by stirring. With respect to the concentration in an aqueous solution to form a self-assembly, because the critical micelle concentration is about 0.5 to about 2 µM (for example, about 1.3 µM, when R¹ is a stearoyl group), a self-assembly can be produced by dissolving the compound of formula I in a concentration not lower than the critical micelle concentration. The higher the concentration of the compound of formula I, the larger the average particle diameter of the self-assembly tends to be. The average particle diameter of the self-assembly of the compound of formula I is preferably about 100 nm or more; and may be, for example, about 100 nm to about 20 µm, preferably about 300 nm to about 10 µm, more preferably about 500 nm to about 7 µm, and particularly preferably about 700 nm to about 5 µm.

The self-assembly as used herein may be obtained by dissolving the compound in water, i.e., in the form of a dispersion in water. Examples of the self-assembly include a wide range of products that can easily reproduce a self-assembly when brought into contact with water, such as lyophilizates thereof.

Examples of the acyl group as used herein includes C₁₄₋₂₄ linear or branched acyl groups that may be substituted with a hydroxy group, such as a myristoyl group, a palmitoyl group, a stearoyl group, an arachidoyl group, a behenoyl group, a lignoceroyl group, a myristoleoyl group, a palmitoleoyl group, an oleoyl group, a linoleoyl group, a γ-linolenoyl group, an α-linolenoyl group, an arachidoyl group, an elaidoyl group, an eicosatrienoyl group, an isostearoyl group, a 12-hydroxystearoyl group, a ricinoleoyl group, and the like.

Such R¹ groups can be obtained by reacting acid chlorides, acid anhydrides, or active esters of corresponding C₁₄₋₂₄, preferably C₁₆₋₂₂, more preferably C₁₆₋₂₀, and particularly C₁₈₋₂₀ linear or branched higher fatty acids that may be substituted with a hydroxy group, such as myristic acid, palmitic acid, stearic acid, arachic acid, behenic acid, lignoceric acid, myristoleic acid, palmitoleic acid, oleic acid, linoleic acid, γ-linolenic acid, α-linolenic acid, arachidonic acid, elaidic acid, eicosatrienoic acid, isostearic acid, and 12-hydroxy-stearic acid, ricinoleic acid and the like, or coupling agents such as DIC (diisopropylcarbodiimide) and DCC (dicyclohexylcarbodiimide) along with corresponding carboxylic acids with the peptide represented by Gly Leu Ala Met Ala Pro Ser Val Gly His Val Arg Gln His Gly-R² (wherein R² is as defined above).

Because the compound of formula I wherein R¹ is H is not capable of forming a self-assembly, the compound exhibits a very weak anti-influenza virus effect, compared to the compound of the present invention. Because the compound of formula I wherein R¹ is a lauryl group (C₁₂) has a low self-assembly-forming capability, the compound exhibits very weak activity in *in vitro* tests. In contrast, a polypeptide wherein R¹ contains 14 carbon atoms (myristoyl) has an intermediate level of activity. A polypeptide wherein R¹ contains 16 to 22 carbon atoms has potent activity. When R¹ contains more than 20 carbon atoms, the obtained peptide has low water solubility. Therefore, R¹ preferably contains 16 to 20 carbon atoms, and particularly 18 to 20 carbon atoms.

Examples of the alkyl group as used herein include alkyl groups containing 1 to 6 carbon atoms, and preferably 1 to 4 carbon atoms, such as methyl, ethyl, n-propyl, isopropyl, n-butyl, isobutyl, sec-butyl, tert-butyl, pentyl, and hexyl. Examples of substituents of the alkyl group include a hydroxy group, a fluorine atom, a methoxy group, an ethoxy group, and the like.

Examples of the aryl group include phenyl, naphthyl, fluorenyl, anthryl, biphenylyl, tetrahydronaphthyl, chromanyl, 2,3-dihydro-1,4-dioxanaphthalenyl, indanyl, and phenanthryl.

Examples of the aralkyl group include benzyl, naphthylmethyl, fluorenylmethyl, anthrylmethyl, biphenylylmethyl, tetrahydronaphthylmethyl, chromanylmethyl, 2,3-dihydro-1,4-dioxanaphthalenylmethyl, indanylmethyl, and phenanthrylmethyl, phenethyl, naphthylethyl, fluorenylethyl, anthrylethyl, biphenylylethyl, tetrahydronaphthylethyl, chromanylethyl, 2,3-dihydro-1,4-dioxanaphthalenylethyl, indanylethyl, and phenanthrylethyl.

Examples of the alkoxy group include O-(alkyl). The alkyl group is as defined above. Examples of the substituent of the alkoxy group include a hydroxy group, a fluorine atom, a methoxy group, an ethoxy group, and the like.

Examples of the aryloxy group include O-(aryl). The aryl group is as defined above.

Examples of the aralkyloxy group include O-(aralkyl). The aralkyl group is as defined above.

Examples of substituents of the alkyl group include a fluorine atom, an alkoxy group, a cyano group, a hydroxy group, and the like.

Examples of substituents of the aryl group, aralkyl group, aryloxy group, or like aryl moiety; and substituents of the aralkyloxy group or like aryl moiety include halogen atoms (F, Cl, Br, I), nitro, amino, monoalkylamino, dialkylamino, acetyl, methylenedioxy, alkyl, alkoxy, carbamoyl, acetylamino, and the like. The number of substituents is 1 to 5, preferably 1 to 3, and more preferably 1 to 2.

R² preferably represents OH, NH₂, NR³R⁴ (wherein R³ and R⁴ are as defined above), or alkoxy.

The compound of formula I as used herein can be obtained by: synthesizing a compound represented by formula I wherein R¹ is a hydrogen atom, and R² is a group as defined above (H-Gly Leu Ala Met Ala Pro Ser Val Gly His Val Arg Gln His Gly-R²) by liquid phase synthesis or solid phase synthesis according to a usual peptide synthesis method, such as the Fmoc method; and introducing an acyl group to R¹ by using a coupling reagent such as DIC. The R² group can be introduced by using Gly-R² as an amino acid on the C-terminus. The R² group can be excised as an OH or NH₂ peptide by suitably selecting a carrier for solid phase synthesis.

Examples of the pharmaceutically acceptable salt of the compound represented by formula I as used herein include inorganic acids such as hydrochloric acid, sulfuric acid, nitric acid, phosphoric acid, and hydrobromic acid; organic acid salts such as trifluoroacetic acid, acetic acid, maleic acid, fumaric acid, malic acid, tartaric acid, methanesulfonic acid, and toluenesulfonic acid; and alkali metal or alkaline earth metal salts such as sodium salts, potassium salts, lithium salts, calcium salts, and magnesium salts.

The self-assembly of the present invention can be formulated into a pharmaceutical preparation comprising the self-assembly of the present invention as an active ingredient, and optionally other components such as pharmacologically acceptable carriers, diluents, and excipients. The pharmaceutical preparation can be administered to, for examples, infected or uninfected humans or other mammals, birds, etc.

The method of administering the prophylactic or therapeutic agent for influenza virus infection according to the present invention is not particularly limited, and can be suitably selected according to the form of the pharmaceutical preparation; the age, sex, and other conditions of the patient; the severity of the disease, etc. Examples of preferable dosage forms are oral preparations such as tablets, capsules, granules, and sublingual tablets; and parenteral dosage forms such as injections, drips, nasal drops, inhalants, patches, and poultices. Inhalants are particularly preferable.

The daily dose of the self-assembly of the compound of formula I used as an active ingredient of the prophylactic or therapeutic agent for influenza according to the present invention (including pharmaceutical compositions and pharmaceutical preparations) may vary depending on the subject's condition, body weight, age, sex, etc., and cannot be completely generalized. However, the dose can typically be selected from the range of about 0.001 to about 100 mg per day per adult. The prophylactic or therapeutic agent for influenza can be administered once a day, or in divided doses.

The present invention is described below in further detail with reference to Reference Examples and Examples, but is not limited thereto.

### Examples

### Reference Example 1

A polypeptide represented by H-Gly Leu Ala Met Ala Pro Ser Val Gly His Val Arg Gln His Gly-NH₂ was synthesized by solid phase synthesis using Fmoc amino acid (1.4 equivalents), HOBt (1-hydroxybenzotriazole; 2.5 equivalents), and DIC (2.8 equivalents) according to the Fmoc method. The obtained polypeptide was allowed to react in a mixed solvent of DMF and DCM using stearic acid (1 equivalent), DIC (2.8 equivalents), and HOBt (2.5 equivalents) under conditions similar to the peptide synthesis for 90 minutes to synthesize a peptide of the present invention represented by (stearoyl)-Gly Leu Ala Met Ala Pro Ser Val Gly His Val Arg Gln His Gly-NH₂ that has a strearoyl group bound to the amino group at the N-terminus (hereinafter referred to as "C18-D1"). C18-D1 was purified by HPLC using a C-18 column. The production of C18-D1 was confirmed by mass spectrometry ([M+H]⁺= 1782.29).

### Reference Example 2

C18-S2 (stearoyl ARLPRTMVHPKPAQP-NH₂), S2 (ARLPRTMVHPKPAQP-NH₂), and D1 (GLAMAPSVGHVRQHG-NH₂) were synthesized in a manner similar to Reference Example 1. The production of C18-S2, S2, and D1 was confirmed by mass spectrometry.

D1 is represented by SEQ ID NO: 1, and S2 is represented by SEQ ID NO: 2.

### Example 1

The critical micelle concentration (CMC) of the purified C18-D1 was determined in the following manner. PBS (pH 7.5) containing 1 µM of N-phenyl-1-naphthylamine (hereinafter abbreviated as "NPN") was prepared. A C18-D1 peptide stock solution (1 mM) was serially diluted to final concentrations of 0.1, 0.3, 1.3, 10, and 30 µM. These solutions were excited by a wavelength of 350 nm, and the fluorescence intensity (FI) at 450 nm was measured. The difference in FI between each diluted peptide solution and PBS alone was plotted as the ordinate, and the peptide solutions were plotted as the abscissa. The concentration at the intersection of the high concentration curve and the low concentration curve was determined. The critical micelle concentration (CMC) of the intersection was 1.3 µM.

### Example 2: In vitro test for the inhibition of influenza infection

Inhibition of influenza virus infection was determined by a plaque assay on MDCK cells. The MDCK cells in 6-well plates were incubated with 0.2 mL of influenza virus A/PR/8/34 solutions (100 to 200 pfu; pfu denotes plaque-forming units; subtype H1N1) or influenza virus A/Victria/1/75 solutions (100-200 pfu; subtype H3N2) each containing C18-D1, S2 (H-ARLPRTMVHPKPAQP-NH₂), C18-S2 (stearoyl-ARLPRTMVHPKPAQP-NH₂), or D1 (H-GLAMAPSVGHVRQHG-NH₂). After incubation at 37°C under 5% CO₂ for 30 minutes, the supernatant was removed, and the cells were washed with PBS. 2 mL (per well) of 2 x MEM + BSA containing 0.6% agarose, 0.01% of O-diethylaminoethyl cellulose dextran, 0.1% NaHO3, and 0.01 µg/mL of acetyl trypsin was added, and the cells were incubated for two days. Viable cells were stained with a crystal violet solution (1 mg/mL in 20% ethanol), and the number of plaques was counted. The maximum infection activity (100%) was defined as the number of plaques in the case of the absence of C18-D1. The IC₅₀ value (50% inhibitory concentration) of C18-D1 was obtained from the plot between log [f/(1-f)] and log [C18-D1], wherein f is the percent infection activity. The IC₅₀ values of S2, C18-S2, and D1 were calculated in a similar manner.

The results of the investigation of influenza virus infection inhibitory effects of compound A on MDCK cells are as follows.

Influenza virus infection inhibitory effect *in vivo* of C18-D1 on MDCK cells was determined by the plaque assay. Infection of MDCK cells with influenza type A virus HA1 (A/PR/8/34 (H1N1)) and influenza type A virus HA3 (A/Victria/1/75 (H3N2)) was inhibited in the presence of C18-D1.

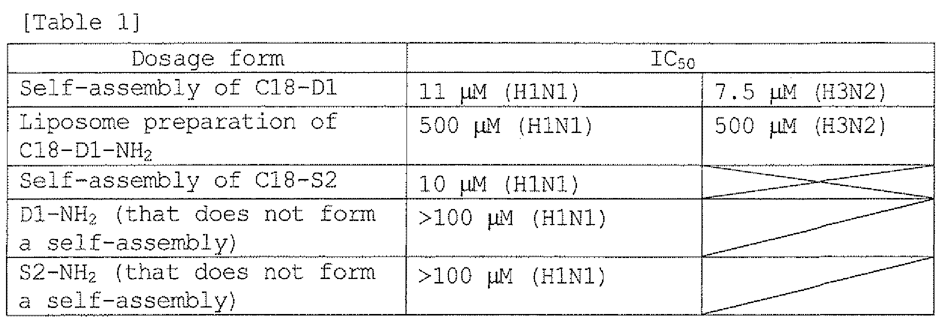

As shown in Table 1, the self-assembly of C18-D1 of the present invention and C18-S2 exhibited excellent anti-influenza virus activity *in vitro*.

### Example 3: Animal test for inhibition of influenza infection

An experiment was conducted to investigate the virus infection inhibitory effect of C18-D1 of the present invention and C18-S2 on mice. Peptide stock solutions (2.5-7.5 mM in PBS), and PBS containing influenza virus (H1N1) (200 pfu) were mixed in the amounts described below, and allowed to stand at room temperature for 30 minutes. The samples thus prepared were administered intranasally to mice (50 µL per mouse).

More specifically, solutions each containing 200 pfu/50 µL of influenza virus, and C18-D1 or C18-S2 in a final concentration of 3.75 mM or a solvent (PBS, mPR8) were administered. When C18-D1 was administered, 80% or more of mice survived (Fig. 1), thus indicating that C18-D1 has infection inhibitory activity. In contrast, administration of C18-S2, which exhibited activity equal to or better than C18-D1 in the *in vitro* test, caused early mortality in the *in vivo* test, even compared to the control.

The results thus show that *in vivo* data of self-assemblies cannot be estimated from *in vitro* data thereof, thus demonstrating an unpredictable effect of the self-assembly of the present invention.

When only the virus (mPR8) was administered, all of the mice died.

The term "mPR8" indicates a control prepared by mixing the influenza virus with PBS.

## Claims

1. A self-assembly of a compound or of a pharmaceutically acceptable salt thereof, the compound being represented by the formula I:
**R¹-Gly Leu Ala Met Ala Pro Ser Val Gly His ValArg Gln His Gly-R²** **(I)**
wherein R¹ represents a linear or branched acyl group having 14 to 24 carbon atoms; R² represents OH, NH₂, NR³R⁴, a substituted or unsubstituted alkoxy group, a substituted or unsubstituted aryloxy group, or a substituted or unsubstituted aralkyloxy group; and R³ and R⁴ independently represent a substituted or unsubstituted alkyl group, a substituted or unsubstituted aryl group, a substituted or unsubstituted aralkyl group, an alkoxy group, or a hydroxy group; provided that both of R³ and R⁴ do not represent a hydroxy group or an alkoxy group simultaneously.

2. The self-assembly according to claim 1 wherein R¹ represents a stearoyl group, a palmitoyl group, an oleoyl group, or a palmitoleyl.

3. The self-assembly according to claim 1 wherein R² represents OH or NH₂.

4. The self-assembly according to claim 1 wherein R¹ represents a stearoyl group, and R² represents OH or NH₂.

5. The self-assembly according to any one of claims 1 to 4 wherein the self-assembly is in the form of a lyophilizate.

6. A prophylactic or therapeutic agent for influenza virus infection comprising the self-assembly of any one of claims 1 to 5.

7. The prophylactic or therapeutic agent according to claim 6 wherein the influenza virus is of subtype H1, H3, H5, or H7.

8. The prophylactic or therapeutic agent according to claim 6 wherein the influenza virus is of subtype H1 or H3.

9. The prophylactic or therapeutic agent according to claim 6 wherein the influenza virus is of subtype H1.

10. A compound, a pharmaceutically acceptable salt thereof, or a self-assembly of the compound or of the pharmaceutically acceptable salt,
the compound being represented by formula I:
**R¹-Gly Leu Ala Met Ala Pro Ser Val Gly His ValArg Gln His Gly-R²** **(I)**
wherein R¹ represents a stearoyl group, and R² represents NH₂.

11. A method of preventing or treating influenza virus infection, comprising administering an effective amount of a compound, a pharmaceutically acceptable salt thereof, or a self-assembly of the compound or of the pharmaceutically acceptable salt to a patient infected with an influenza virus or a subject who is at risk for infection,
the compound being represented by formula I:
**R¹-Gly Leu Ala MetAla Pro Ser Val Gly His ValArg Gln His Gly-R²** **(I)**
wherein R¹ represents a linear or branched acyl group having 14 to 24 carbon atoms; R² represents OH, NH₂, NR³R⁴, a substituted or unsubstituted alkoxy group, a substituted or unsubstituted aryloxy group, or a substituted or unsubstituted aralkyloxy group; and R³ and R⁴ independently represent a substituted or unsubstituted alkyl group, a substituted or unsubstituted aryl group, a substituted or unsubstituted aralkyl group, an alkoxy group, or a hydroxy group; provided that both of R³ and R⁴ do not represent a hydroxy group or an alkoxy group simultaneously.


SEQUENCE LISTING

<110> KEIO UNIVERSITY

<120> Anti-influenza virus agent

<130> 147 466 n2/n17

<140> 09.809762.9
<141> 2009-8-07

<150> JP 2008-222062
<151> 2008-8-29

<160> 2

<170> PatentIn version 3.4

<210> 1
<211> 15
<212> PRT
<213> Artificial

<220>
<223> Sequence

<400> 1

Gly Leu Ala Met Ala Pro Ser Val Gly His Val Arg Gln His Gly
1               5                   10                  15


<210> 2
<211> 15
<212> PRT
<213> Artificial

<220>
<223> Sequence

<400> 2

Ala Arg Leu Pro Arg Thr Met Val His Pro Lys Pro Ala Gln Pro
1               5                   10                  15